# EUROPÄISCHE PATENTANMELDUNG

(11) **EP 1 060 730 A2**
(43) Veröffentlichungstag der Anmeldung: **20.12.2000**
(21) Anmeldenummer: 00112469.2
(22) Anmeldetag: 10.06.2000
(51) Int. Cl.: A61J 1/14

(54) **Konnektoranordnung mit einem Anschlusssystem und einem Portsystem**

(30) Priorität: 16.06.1999 DE 19927356
(71) Anmelder: Fresenius Kabi Deutschland GmbH, 61352 Bad Homburg v.d.H. (DE)
(72) Erfinder: Knierbein, Bernd, Dr., 61267 Neu-Anspach (DE)
(74) Vertreter: Luderschmidt, Schüler & Partner GbR

(57) **Zusammenfassung**

Eine Konnektoranordnung besteht aus einem Anschlußsystem (2) und einem Portsystem (1) zum Anschluß einer Schlauchleitung (17) an eine Verpackung, die eine Flüssigkeit für medizinische Anwendungen enthält. Das Anschlußsystem (2) umfaßt einen Spike (3), an den die Schlauchleitung (17) anschließbar ist, und eine den Spike über seine gesamte Länge umschließende Abdeckung (4). Die Spikeabdeckung (4) besteht aus zwei Teilstücken (18, 19), die zur teleskopartigen Verlängerung oder Verkürzung miteinander verschraubbar sind. Zur Konnektierung des Anschlußsystems (2) an das Portsystem (1) wird die Spikeabdeckung (4) an das Portsystem (1) konnektiert. Anschließend wird das erste Teilstück (18) vollständig auf das zweite Teilstück (19) aufgeschraubt, wodurch sich der Spike (3) vorschiebt und die Membran (14) durchstößt.

## Beschreibung

Die Erfindung betrifft eine Konnektoranordnung mit einem Anschlußsystem und einem Portsystem zum Anschluß einer Schlauchleitung an eine Verpackung, die eine Flüssigkeit für medizinische Anwendungen enthält.

Die DE-A-197 17 765 beschreibt eine sterile Konnektoranordnung zum Anschluß einer Schlauchleitung an einen Folienbeutel, der eine Flüssigkeit für medizinische Anwendungen enthält. Die bekannte Konnektoranordnung verfügt über ein Anschlußstück, das mit einer kanalförmigen Ausnehmung versehen ist, die von einer durchstechbaren Membran verschlossen ist. Das Anschlußstück besteht aus einem mit dem Beutel verschweißbaren Unterteil und einem rohrförmigen Oberteil zur Aufnahme eines Einstechdorns (Spike), an dem die Schlauchleitung angeschlossen ist. Eine sterile Konnektoranordnung der oben beschriebenen Art ist auch aus der DE-A- 196 37 856 bekannt.

Die bekannten Konnektoranordnungen haben sich in der Praxis bewährt. Nachteilig ist jedoch, daß der Spike vor dem Anschluß an den Beutel der nichtsterilen Umgebung ausgesetzt ist. Als Berührungsschutz sind Kappen oder dgl. bekannt, nachteilig ist aber, daß diese vor dem Einschieben des Spike in das Anschlußstück abgenommen werden müssen, so daß der Spike beim Einschieben ungeschützt bleibt.

Aus der PCT96/00093 ist eine Konnektoranordnung bekannt, deren Spike von einem flexiblen Schlauch umschlossen ist, der mit einer Überwurfmutter an dem rohrförmigen Oberteil des Anschlußstücks befestigt wird. Zur Herstellung der Flüssigkeitsverbindung wird der Spike in das rohrförmige Oberteil des Anschlußstücks eingeschoben, wodurch die das Oberteil verschließende Membran durchstochen wird. Nachteilig ist, daß die maximale Dehn- und Stauchbarkeit des Schlauchs der Verschiebbarkeit des Spike Grenzen setzt. Dies ist insofern nachteilig, als eine tief in dem Anschlußstück liegende Membran, die ausreichend gegen Berühren geschützt ist, nur dann von dem Spike durchstochen werden kann, wenn dieser tief in das Anschlußstück eingeschoben wird, was die Handhabung erschwert. Des weiteren besteht die Gefahr, daß der flexible Schlauch reißt, was eine Undichtigkeit des Systems zur Folge hat. Darüber hinaus erschwert das Aufziehen des flexiblen Schlauchs auf den Spike die Herstellung der Konnektoranordnung. Angesichts der Entsorgung ist auch nachteilig, daß der Konnektor aus unterschiedlichen Materialien besteht.

Der Erfindung liegt daher die Aufgabe zugrunde, eine leicht zu handhabende und ohne großen Aufwand einfach herzustellende Konnektoranordnung zu schaffen, deren Spike beim Anschluß an die Verpackung gegen Berühren ausreichend geschützt ist.

Die Lösung dieser Aufgabe erfolgt erfindungsgemäß mit den im Patentanspruch 1 bzw. 9 angegebenen Merkmalen.

Der Spike der erfindungsgemäßen Konnektoranordnung wird über seine gesamte Länge von einer Spikeabdeckung umschlossen, die ein erstes Teilstück und ein zweites Teilstück aufweist. Zur teleskopartigen Verlängerung oder Verkürzung der Spikeabdeckung sind das erste und zweite Teilstück miteinander verschraubbar. Hierzu kann entweder das erste Teilstück mit einem Innen- und das zweite Teilstück mit einem Außengewinde oder das erste Teilstück mit einem Außen- und das zweite Teilstück mit einem Innengewinde versehen sein. Das zweite Teilstück der Spikeabdeckung ist an das Konnektorstück des Portsystems konnektierbar.

Zum Anschluß des Spikes an das Portsystem wird die Spikeabdeckung an das Konnektorstück konnektiert. Anschließend wird zur axialen Verkürzung der Spikeabdeckung das erste Teilstück vollständig auf bzw. in das zweite Teilstück auf bzw. eingeschraubt, wodurch der Spike die Membran durchstößt, so daß die Flüssigkeitsverbindung hergestellt wird. Dabei bleibt der Spike gegen Berühren geschützt.

In einer bevorzugten Ausführungsform ist die Membran eine an der Unterseite des Einsatzstücks angeordnete Folie. Die durchstechbare Membran kann aber auch eine zwischen Konnektorstück und Einsatzstück angeordnete Folie sein. Als Membran können anstelle einer Folie auch Kunststoffplatten oder dgl. Verwendung finden, die mit dem Portsystem einstückig sind. Je tiefer die Membran liegt, desto länger müssen das erste und zweite Teilstück der Spikeabdeckung sein. Mit einer höher liegenden Membran kann also die axiale Länge der Spikeabdeckung verkürzt werden. Ist die Membran in der Mitte der kanalförmigen Ausnehmung angeordnet, wird ein kompakter Aufbau bei ausreichendem Berührungsschutz der Membran erzielt.

Das zweite Teilstück der Spikeabdeckung wird zweckmäßigerweise mit dem Konnektorstück verschraubt. Um das zweite Teilstück auf das Konnektorstück aufschrauben zu können, ist an dem Konnektorstück ein Außen- und an dem zweiten Teilstück ein Innengewinde vorgesehen. Anstelle eines Schraubverschlusses kann aber auch ein Bajonettverschluß oder dgl. vorgesehen sein.

Bei einer weiteren vorteilhaften Ausführung bildet das zweite Teilstück der Spikeabdeckung einen Anschlag für das ersten Teilstück, in dem das zweite Teilstück aus zwei Abschnitten mit unterschiedlichen Durchmessern besteht. Zur unverlierbaren Sicherung des zweiten Teilstücks der Spikeabdeckung kann ein Gewindeanschlag in dem ersten Teilstück vorgesehen sein. Ferner sollte das erste Teilstück an dem Spike unverlierbar befestigt sein.

Damit sich der Spike beim Anschluß an des Portsystem nicht mitdreht, ist an dem Konnektorstück vorteilhafterweise ein Führungsstück vorgesehen, in das der Spike verdrehsicher einschiebbar ist.

Eine alternative Ausführungsform der Konnektoranordnung verzichtet auf die teleskopartige Verlängerung der Spikeabdeckung. Die Spikeabdeckung ist eine Überwurfmutter, die sich über die Spitze des Spikes hinaus erstreckt, so daß der Spike über seine gesamte Länge umschlossen ist. Bei dieser Ausführungsform ist die Membran nicht in dem Einsatzstück oder zwischen Einsatzstück und Konnektorstück, sondern im Konnektorstück des Portsystems angeordnet, so daß der Spike die hochliegende Membran beim Aufschrauben der Spikeabdeckung durchstoßen kann.

Im folgenden werden mehrere Ausführungsbeispiele der Erfindung unter Bezugnahme auf die Zeichnungen näher erläutert.

Es zeigen:
- Figur 1: Ein erstes Ausführungsbeispiel des Anschluß- und Portsystems der Konnektoranordnung mit einer tiefliegenden Membran in geschnittener Darstellung,
- Figur 2: das Anschlußsystem der Konnektoranordnung in geschnittener Darstellung von Figur 1,
- Figur 3: ein zweites Ausführungsbeispiel der Konnektoranordnung, die einen besonders kompakten Aufbau hat, und
- Figur 4: ein drittes Ausführungsbeispiel der Konnektoranordnung in geschnittener Darstellung, deren Spikeabdeckung nicht teleskopartig verlängerbar ist.

Die Konnektoranordnung besteht aus einem Portsystem 1 und einem Anschlußsystem 2, das sich aus einem Spike 3 und einer Spikeabdeckung 4 zusammensetzt.

Das als Spritzgießteil aus Polypropylen hergestellte Portsystem 1 weist ein nach Art eines Schiffchens ausgebildetes Einsatzstück 5 mit einem rohrförmigen Abschnitt 6 auf, der mit zwei radial abstehenden Ansätzen 7, 8 versehen ist, die in einer Ebene liegen. Das Einsatzstück kann in die bekannten Verpackungen, die eine Flüssigkeit für medizinische Anwendungen enthalten, eingesetzt werden. Das Einsatzstück kann beispielsweise in Folienbeutel eingeschweißt werden, die mit einer enteralen Nährlösung befüllt sind.

An das Einsatzstück 5 des Portsystems 1 schließt sich ein rohrförmiges Konnektorstück 9 an, das mit einem Außengewinde 10 zum Aufschrauben der Spikeabdeckung 4 des Anschlußsystems 2 versehen ist. An seinem oberen Rand weist das Konnektorstück 9 einen umlaufenden, nach innen vorspringenden Ansatz 11 auf, der in ein Führungstück 12 zur verdrehsicheren Aufnahme des Spike 3 des Anschlußsystems 2 übergeht. Das Führungsstück 12 weist einen rechteckförmigen Querschnitt mit abgerundeten Ecken auf, der dem Querschnitt des Spike 3 entspricht.

Die kanalförmige Ausnehmung 13 in dem Einsatzstück 5 und dem Konnektorstück 9 ist mit einer Membran 14 verschlossen. Die Membran 14 ist eine mit der Unterseite des Einsatzstücks verschweißte Folie mit sehr geringer Sauerstoffdurchlässigkeit, die sich nahe zu über die gesamte untere Fläche des Anschlußstücks erstreckt. Ein derartiges Portsystem ist in der DE-A-197 17 765 im einzelnen beschrieben, auf die ausdrücklich Bezug genommen wird.

Der Spike 3 des Portsystems 2 weist an seinem unteren Ende eine Spitze 15 auf, an seinem oberen Ende verfügt der Spike über ein Verbindungsstück 16 zum Anschluß einer Schlauchleitung 17 eines nicht dargestellten Schlauchleitungssystems. Der Spike 3 dichtet gegenüber dem inneren Führungsstück 12 des Konnektorstücks 9 ab.

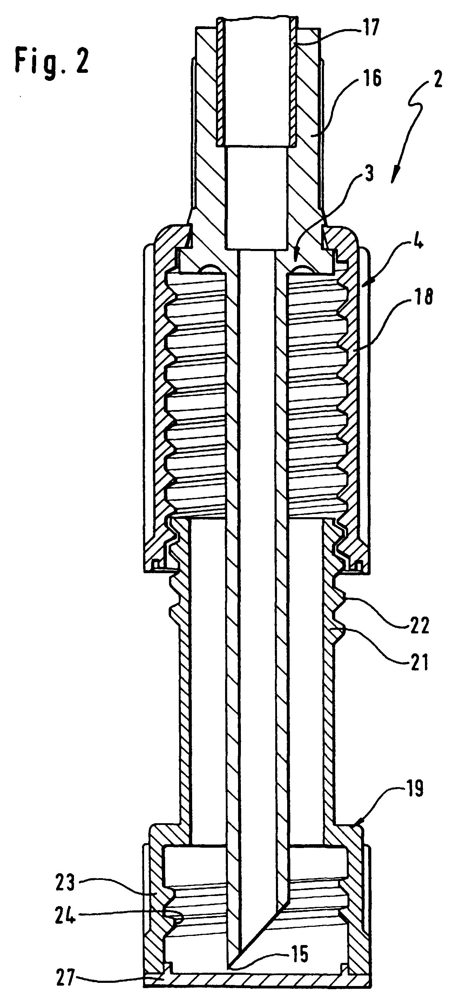

Die Spikeabdeckung 4 besteht aus einem ersten rohrförmigen Teilstück 18 und einem zweiten rohrförmigen Teilstück 19, die miteinander verschraubt sind. Das erste Teilstück 18 der Spikeabdeckung 4 ist drehbar, aber unverlierbar an dem Verbindungsstück 16 des Spikes 3 befestigt. Es erstreckt sich bis etwa über die halbe Länge des Spike und weist ein Innengewinde 20 auf. Das zweite Teilstück 19 weist einen ersten Abschnitt 21 mit einem Außengewinde 22, der mit dem ersten Teilstück 18 der Spikeabdeckung 4 verschraubbar ist, und einen zweiten Abschnitt 23 mit einem Innengewinde 24 auf, der mit dem Konnektorstück 9 des Portsystems 1 verschraubbar ist. Der zweite Abschnitt 23 des zweiten Teilstücks 19 weist einen größeren Durchmesser als der erste Abschnitt 21 auf, so daß das zweite Teilstück einen Anschlag für das erste Teilstück 20 der Spikeabdeckung 4 bildet. Die Länge des ersten und zweiten Teilstücks der Spikeabdeckung ist so bemessen, daß der Spike über seine gesamte Länge von der Spikeabdeckung umschlossen wird, wenn die beiden Teilstücke nicht vollständig miteinander verschraubt sind. Zur Erhöhung der Sicherheit kann die Spikeabdeckung 4 mit einer zusätzlichen Abdeckkappe 27 verschlossen werden, die einrastend in das zweite Teilstück 19 der Spikeabdeckung 4 eingesetzt wird (Figur 2).

Zur Konnektierung des Anschlußsystems 2 an das Portsystem 1 wird das zweite Teilstück 19 der Spikeabdeckung 4 mit dem Konnektorstück 9 des Portsystems 1 verschraubt. Dabei stützt sich das zweite Teilstück 19 der Spikeabdeckung 4 an einem ringförmigen Ansatz 26 des Konnektorstücks 9 ab. Anschließend wird das erste Teilstück 18 vollständig auf das zweite Teilstück 19 der Spikeabdeckung 4 geschraubt, wodurch sich der Spike 3 in das Führungsstück 12 des Konnektorstücks 9 und weiter in die kanalförmige Ausnehmung 13 des Portsystems vorschiebt, bis er die Membran 14 durchstößt. Damit ist eine Flüssigkeitsverbindung hergestellt.

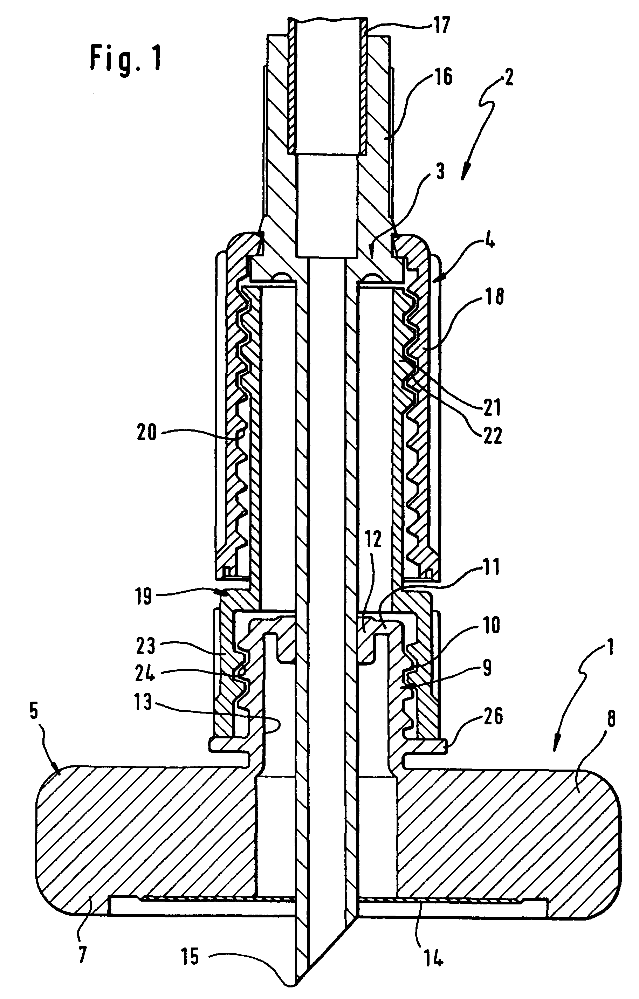

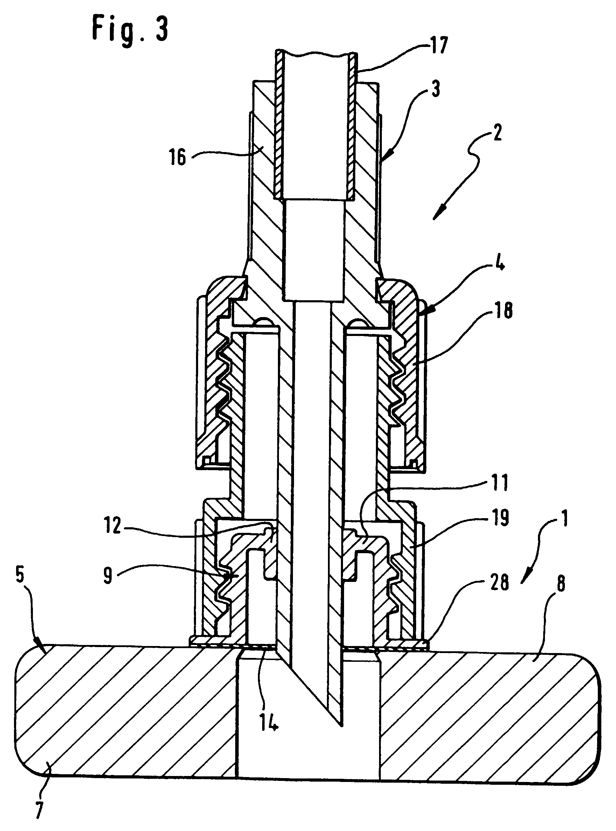

Figur 3 zeigt ein Ausführungsbeispiel der Konnektoranordnung, die einen kompakteren Aufbau hat. Die Teile der Ausführungsform von Figur 3, die denjenigen des Ausführungsbeispiels von den Figuren 1 und 2 entsprechen, sind mit den gleichen Bezugszeichen versehen. Die Ausführungsform von Figur 3 unterscheidet sich von dem Ausführungsbeispiel gemäß der Figuren 1 und 2 lediglich dadurch, daß die Membran 14 nicht eine mit der Unter-, sondern der Oberseite des Einsatzstücks 5 des Portsystems 1 verschweißte Folie ist. Das Konnektorstück 9 ist bei dieser Ausführungsform mit einem ringförmigen Flansch 28 versehen, der mit dem Einsatzstück 5 verschweißt ist. Da die Membran 14 hochgelegt ist, kann die Länge des ersten und zweiten Teilstücks 18, 19 der Spikeabdeckung 4 verkürzt werden, wodurch die Konnektoranordnung einen kompakten Aufbau hat.

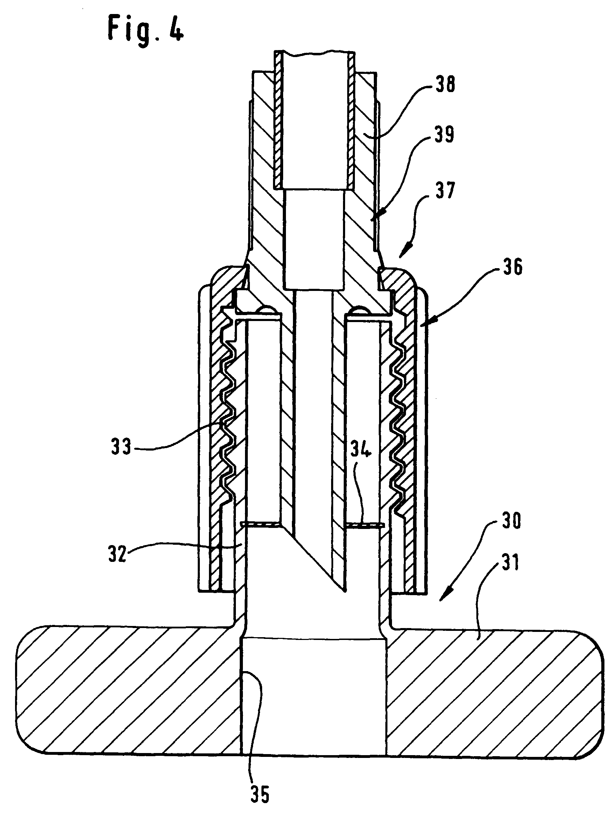

Figur 4 zeigt ein Ausführungsbeispiel einer Konnektoranordnung mit einem vereinfachten Aufbau, die sich von den oben beschriebenen Ausführungsbeispielen dadurch unterscheidet, daß die Spikeabdeckung nicht teleskopartig verlängerbar ist. Das Portsystem 30 der Konnektoranordnung weist ein Einsatzstück 31 mit einem rohrförmigen Konnektorstück 32 auf, das mit einem Außengewinde 33 versehen ist. Die Membran 34 zum Verschließen der kanalförmigen Ausnehmung 35 des Portsystems 30 ist in der unteren Hälfte des Konnektorstücks 33 angeordnet. Die Membran 34 kann ein in das Konnektorstück eingesetztes Spritzgießteil oder mit dem Konnektorstück einteilig spritzgegossen sein. Die Spikeabdeckung 36 des Anschlußsystems 37 ist eine Überwurfmutter, die an dem Verbindungsstück 38 des Spike 39 unverlierbar gesichert ist. Sie erstreckt sich über die gesamte Länge des Spike.

Zur Konnektierung des Anschlußsystems 37 an das Portsystem 30 wird die Überwurfmutter 36 auf das Konnektorstück geschraubt, wodurch der Spike 39 in das Konnektorstück 33 vorgeschoben und die Membran 34 durchstoßen wird. Damit ist die Flüssigkeitsverbindung hergestellt.

## Patentansprüche

1. Konnektoranordnung mit einem Anschlußsystem (2) und einem Portsystem (1) zum Anschluß einer Schlauchleitung an eine Verpackung, die eine Flüssigkeit für medizinische Anwendungen enthält, wobei
das Anschlußsystem (2) einen Spike (3), an den die Schlauchleitung anschließbar ist, und eine den Spike über seine gesamte Länge umschließende Abdeckung (4) umfaßt, und
das Portsystem (1) ein in die Verpackung einsetzbares Einsatzstück (5) umfaßt, das eine kanalförmige Ausnehmung (13) aufweist, die sich in einem an das Einsatzstück anschließenden Konnektorstück (9) fortsetzt, wobei die kanalförmige Ausnehmung (13) mit einer durchstechbaren Membran (14) verschlossen ist,
dadurch gekennzeichnet,
daß die Spikeabdeckung (4) des Anschlußsystem (2) ein erstes Teilstück (18) und ein zweites Teilstück (19) aufweist, die zur Verlängerung oder Verkürzung miteinander verschraubbar sind, wobei das zweite Teilstück (19) an das Konnektorstück (9) des Portsystems (1) konnektierbar ist.

2. Konnektoranordnung nach Anspruch 1, dadurch gekennzeichnet, daß die durchstechbare Membran (14) eine an der dem Konnektorstück (9) abgewandten Seite des Einsatzstücks (5) angeordnete Folie ist.

3. Konnektoranordnung nach Anspruch 1, dadurch gekennzeichnet, daß die durchstechbare Membran (14) eine zwischen dem Konnektorstück (9) und dem Einsatzstück (5) angeordnete Folie ist.

4. Konnektoranordnung nach einem der Ansprüche 1 bis 3, dadurch gekennzeichnet, daß das Konnektorstück (9) ein Außengewinde (10) und das zweite Teilstück (19) der Spikeabdeckung (4) ein Innengewinde (24) aufweist, so daß die Spikeabdeckung auf das Konnektorstück aufschraubbar ist.

5. Konnektoranordnung nach Anspruch 4, dadurch gekennzeichnet, daß das erste Teilstück (18) der Spikeabdeckung (4) eine Überwurfmutter mit einem Innengewinde (20) ist, wobei das zweite Teilstück (19) ein Außengewinde (22) aufweist, so daß das erste Teilstück (18) auf das zweite Teilstück (19) aufschraubbar ist.

6. Konnektoranordnung nach Anspruch 5, dadurch gekennzeichnet, daß das zweite Teilstück (19) der Spikeabdeckung (4) einen ersten Abschnitt (21) mit einem kleineren und einem zweiten Abschnitt (23) mit einem größeren Durchmesser aufweist, so daß das zweite Teilstück einen Anschlag für das erste Teilstück bildet.

7. Konnektoranordnung nach einem der Ansprüche 1 bis 6, dadurch gekennzeichnet, daß das erste Teilstück (18) der Spikeabdeckung (4) an dem Spike (3) drehbar befestigt ist.

8. Konnektoranordnung nach einem der Ansprüche 1 bis 7, dadurch gekennzeichnet, daß der Spike (3) verdrehsicher in einem Führungsstück (12) einschiebbar ist, das an dem Konnektorstück (9) vorgesehen ist.

9. Konnektoranordnung mit einem Anschlußsystem (37) und einem Portsystem (30) zum Anschluß einer Schlauchleitung an eine Verpackung, die eine Flüssigkeit für medizinische Anwendungen enthält, wobei
das Portsystem (30) ein in die Verpackung einsetzbares Einsatzstück (31) umfaßt, das eine kanalförmige Ausnehmung (35) aufweist, die sich in einem an das Einsatzstück anschließenden Konnektorstück (32) fortsetzt, wobei die kanalförmige Ausnehmung mit einer durchstechbaren Membran (34) verschlossen ist, und
das Anschlußsystem (37) einen Spike (39), an den die Schlauchleitung anschließbar ist, und eine Überwurfmutter (36) umfaßt, die auf das Konnektorstück (32) aufschraubbar ist,
dadurch gekennzeichnet, daß die Überwurfmutter (36) als eine den Spike (39) über seine gesamte Länge umschließende Abdeckung ausgebildet ist, wobei die Membran (34) derart in dem Konnektorstück (33) angeordnet ist, daß diese beim Aufschrauben der Überwurfmutter auf das Konnektorstück zur Herstellung der Flüssigkeitsverbindung durchstochen wird.

10. Konnektoranordnung nach Anspruch 9, dadurch gekennzeichnet, daß die Überwurfmutter(36) unverlierbar an dem Spike (39) befestigt ist.
